# FASCICULE DE BREVET EUROPEEN

(11) **EP 3 400 059 B1**
(45) Date de publication et mention de la délivrance du brevet: **08.03.2023**
(21) Numéro de dépôt: 17700260.7
(22) Date de dépôt: 06.01.2017
(51) Int. Cl.: A61N 5/06, A61B 18/20, A61B 18/00

(54) **DISPOSITIF DE TRAITEMENT DERMATOLOGIQUE ÉQUIPÉ D'UN MOYEN DE SURVEILLANCE DES VENTILATEURS**
DERMATOLOGISCHE BEHANDLUNGSVORRICHTUNG MIT VORRICHTUNG ZUR ÜBERWACHUNG VON LÜFTERN
DERMATOLOGICAL TREATMENT DEVICE PROVIDED WITH A MEANS FOR MONITORING FANS

(30) Priorité: 07.01.2016 FR 1600035
(43) Date de publication de la demande: 14.11.2018
(73) Titulaire: Urgo Recherche Innovation et Développement, 21300 Chenove (FR)
(72) Inventeur: LAMOISE, Michel, 21110 Bessey Les Cîteaux (FR); LE LOUS, Guirec, 75007 Paris (FR); PEYRE, Jean-François, 13790 Rousset (FR)
(74) Mandataire: Barbot, Willy
(86) Numéro de dépôt international: PCT/EP2017/000013
(87) Numéro de publication internationale: WO 2017/118606

(56) Documents cités:
- WO-A1-2009/071592
- GB-A- 2 421 982
- US-A1- 2012 283 712
- US-B1- 6 407 672

## Description

La présente invention concerne un dispositif de traitement dermatologique comprenant une tête laser apte à tirer un faisceau laser. Un tel dispositif est typiquement employé pour créer un échauffement précis et localisé d'une zone cible, laquelle zone cible correspond à la plaie d'un patient qui comprend des tissus dermiques et ceci afin d'en accélérer la cicatrisation.

Un tel dispositif de traitement dermatologique est par exemple illustré par le dispositif décrit dans la demande internationale WO 2009/071592 de la Demanderesse. Un autre dispositif de traitement dermatologique comprenant une tête laser est connu du document US 2012/283712 A1.

Maintenant, si un échauffement de la zone cible est souhaité, ce n'est pas le cas d'un échauffement du dispositif de traitement dermatologique en tant que tel. En effet, un tel échauffement pourrait s'avérer préjudiciable, tant à la performance qu'à la longévité d'un tel dispositif. Aussi, et afin de limiter un tel échauffement, un tel dispositif de traitement dermatologique est typiquement équipé d'au moins un ventilateur, lequel est disposé et configuré de manière à dissiper la chaleur dégagée par la tête laser.

En lien avec cet échauffement, il est bien évidemment de nature à conduire à un dysfonctionnement de la tête laser s'il est trop important, mais pas seulement. En effet, lorsqu'il s'agit de contrôler la quantité de chaleur transmise aux tissus, le dispositif comprend généralement un pyromètre apte à mesurer la température desdits tissus. Dès lors, un échauffement trop important de la tête laser pourrait, de manière préjudiciable, fausser la température mesurée par le pyromètre et ainsi impacter défavorablement la quantité de chaleur transmise aux tissus, soit en entraînant une brûlure de ces derniers, soit en leur administrant un traitement inefficace.

Aussi est-il important de surveiller toutes les causes possibles d'un tel échauffement en vue de le prévenir.

Pour cela la présente invention propose un dispositif de traitement dermatologique comprenant une tête laser apte à tirer un faisceau laser en direction d'une zone cible, au moins un ventilateur apte à dissiper la chaleur dégagée par la tête laser, et au moins un moyen de surveillance apte à détecter un défaut d'au moins un ventilateur et à commander au moins un moyen d'inhibition du faisceau laser lorsqu'un tel défaut est détecté selon la revendication 1.

Selon une autre caractéristique, la commande du moyen d'inhibition est persistante.

Le moyen de surveillance observe un signal indicatif de la vitesse dudit au moins un ventilateur et détecte un défaut lorsque la vitesse devient inférieure à un seuil de vitesse ou s'annule.

Le signal indicatif de la vitesse est le courant de commande circulant dans ledit au moins un ventilateur. Selon une autre caractéristique, le seuil de vitesse est configurable. Selon une autre caractéristique, le moyen de surveillance est encore apte à envoyer une alerte à l'opérateur lorsqu'un défaut est détecté.

Le moyen de surveillance est câblé prenant la forme d'un circuit électrique et/ou électronique. Selon une autre caractéristique, le moyen de surveillance comprend au moins un composant TC670, produit par MICROCHIP, préférentiellement un tel composant par ventilateur.

Le dispositif comprend encore au moins un pyromètre à proximité de la tête laser et apte à mesurer une température de la zone cible tel que décrit dans le brevet EP 2,237,731. Un tel pyromètre est séparé de la tête laser par un isolant thermique, préférentiellement une mousse.

D'autres caractéristiques, détails et avantages de l'invention ressortiront plus clairement de la description détaillée donnée ci-après à titre indicatif en relation avec des dessins sur lesquels :
- la figure 1 présente schématiquement un dispositif de traitement dermatologique dans son environnement d'utilisation,
- la figure 2 présente le câblage d'un composant de surveillance.

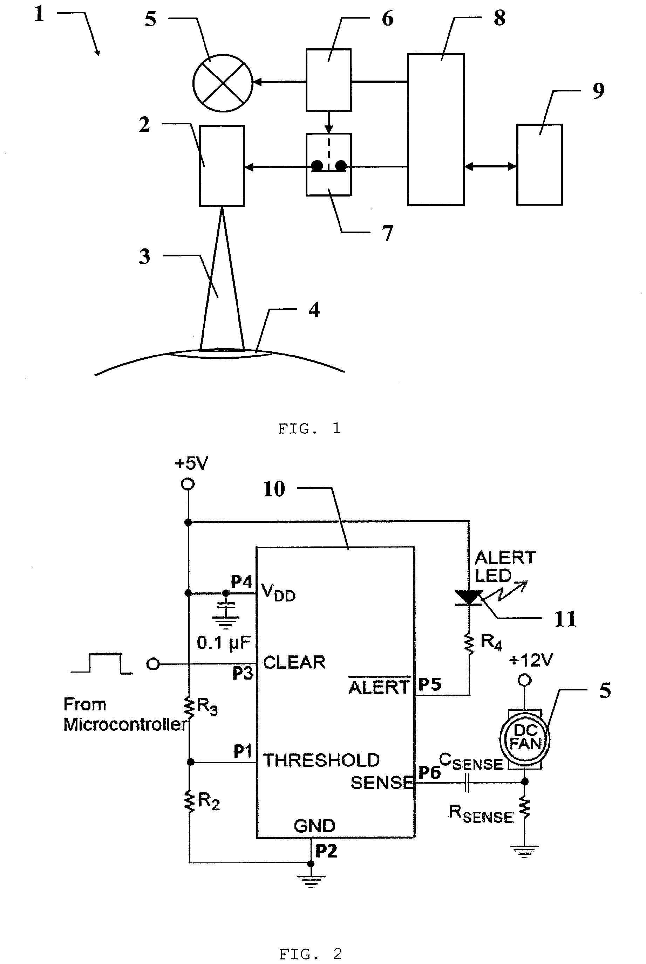

Tel qu'illustré à la figure 1, le dispositif 1 de traitement dermatologique comprend une tête laser 2. La tête laser 2 est apte à tirer un faisceau laser 3 en direction d'une zone cible 4, située sur la peau d'un patient. Cette illumination a pour but de produire un échauffement contrôlé de la peau au niveau de la zone cible 4, lequel échauffement permet de favoriser la cicatrisation.

Une unité de commande 8 permet le pilotage du dispositif 1 et commande ainsi la tête laser 2 et le ou les ventilateurs 5. Une interface homme machine 9 permet à un opérateur d'échanger avec le dispositif 1 et particulièrement l'unité de commande 8 afin de configurer et de commander le dispositif 1.

De manière parasite, le fonctionnement de la tête laser 2 pour réaliser des tirs laser provoque son échauffement et celui des composants se trouvant à proximité. Aussi, au moins un ventilateur 5 est avantageusement disposé de manière à dissiper la chaleur dégagée par cette tête laser 2.

Compte tenu des conséquences, pouvant être graves, d'un échauffement trop important causé par un mauvais fonctionnement d'au moins un ventilateur 5, une surveillance des ventilateurs 5 est particulièrement avantageuse.

Pour cela, le dispositif 1 de traitement dermatologique comprend encore au moins un moyen de surveillance 6 apte à détecter un défaut d'au moins un ventilateur 5 et à commander au moins un moyen d'inhibition 7 du faisceau laser 3 lorsqu'un défaut est détecté.

Ainsi, dès qu'un dysfonctionnement d'au moins un desdits au moins un ventilateur 5 est détecté, ledit au moins un moyen d'inhibition 7 est commandé afin de stopper immédiatement tout tir laser et toute conséquence possible du faisceau laser 3. Une inhibition du faisceau laser 3 signifie qu'un tir en cours est immédiatement stoppé et qu'un tir futur est rendu impossible. Une demande de déclenchement d'un nouveau tir est inopérante en ce qu'une demande de tir est impossible ou refusée.

Le moyen d'inhibition 7 peut agir de différentes manières, tant qu'il empêche le faisceau laser 3 d'atteindre la zone cible 4. Le moyen d'inhibition 7 peut être électrique et/ou logique et intervenir en amont en coupant la puissance d'alimentation du laser ou de la tête laser 2, ou en coupant sa commande. Le moyen d'inhibition 7 peut encore être optique en ce qu'il modifie ou occulte le faisceau laser 3 en déplaçant une lentille optique présente sur l'axe du faisceau laser 3 ou en ce qu'il intercale un élément occultant dans le faisceau laser 3, tel un écran, entre la tête laser 2 et la cible 4.

L'interdiction d'un nouveau tir peut encore comprendre un blocage mécanique, électrique ou logique, d'un moyen de commande de tir.

L'inhibition du faisceau laser 3 doit avantageusement être prolongée jusqu'à ce qu'une analyse du défaut l'ayant causée puisse être pratiquée, et ce même si la détection du défaut par le moyen de surveillance 6 produit un signal sporadique. Aussi, selon une caractéristique avantageuse, la commande du moyen d'inhibition 7 est persistante. Le moyen d'inhibition 7, une fois déclenché dans un état inhibé par une alerte de défaut issue du moyen de surveillance 6, reste dans cet état inhibé, et ceci même en cas de disparition de l'alerte de défaut. Ceci est réalisé en fonction de la technologie employée pour le moyen de surveillance 6 et le moyen d'inhibition 7. Ainsi, ce peut être une mémoire pour une technologie informatique, ou encore un dispositif d'auto-entretien pour une technologie à base de relais. Le dispositif assurant la persistance peut être intégré au moyen de surveillance 6, au moyen d'inhibition 7 ou encore à l'unité de commande 8.

Une action de réarmement doit avantageusement être réalisée, par une personne qualifiée, une fois le défaut corrigé, pour lever l'inhibition et permettre à nouveau de réaliser un tir laser.

Le moyen de surveillance 6 observe un signal indicatif de la vitesse dudit au moins un ventilateur 5. Le moyen de surveillance 6 détecte un défaut lorsque la vitesse devient inférieure à un seuil de vitesse ou s'annule. Le moyen de surveillance 6 émet alors une alerte signalant ledit défaut.

Selon un mode de réalisation possible non revendiqué, le signal indicatif de la vitesse est un signal issu d'un capteur de vitesse entraîné par le ventilateur 5.

Selon le mode de réalisation de l'invention, avantageux en ce qu'il ne nécessite ni composant, ni câblage supplémentaire, le signal indicatif de la vitesse est le courant de commande circulant dans ledit au moins un ventilateur 5. Du fait du type de moteur utilisé par les ventilateurs 5, le courant de commande d'un ventilateur 5 est représentatif de la vitesse effectivement atteinte par le ventilateur 5. Aussi l'observation de ce courant permet d'obtenir une estimation de la vitesse du ventilateur 5.

Selon une autre caractéristique, le seuil de vitesse est avantageusement configurable. Ceci permet avantageusement de placer un tel seuil de vitesse en dessous de la vitesse minimale généralement suivie par le ventilateur 5. Ainsi, si la vitesse réelle du ventilateur 5 descend en dessous de ce seuil de vitesse, il est certain ou du moins fort probable que le ventilateur présente un défaut.

Une vitesse nulle ou un ventilateur arrêté, quelle qu'en soit la raison, est un cas particulier d'une vitesse trop faible, éventuellement détectable par d'autres moyens que l'observation de la vitesse.

La première fonction du moyen de surveillance 6 est, lorsqu'il détecte un défaut, de transmettre une alerte, par tout moyen, à l'attention d'un moyen d'inhibition 7 afin de commander un passage de ce dernier dans un état inhibé. En plus de cette fonction principale, le moyen de surveillance 6 peut encore avantageusement, directement ou via au moins un moyen intermédiaire, envoyer une alerte à l'opérateur afin de l'informer qu'un défaut a été détecté. Cette alerte peut être transmise par tout moyen et sous différentes modalités. Il peut s'agir d'un avertisseur visible ou sonore. Il peut encore s'agir d'une information transmise à une interface homme machine 9 afin que cette dernière affiche l'alerte à l'attention de l'opérateur. Une telle transmission d'alerte est utile en ce qu'elle permet à l'opérateur de comprendre pourquoi le faisceau laser est inhibé, soit que le tir en cours s'arrête, soit qu'une tentative de débuter un tir ne soit pas suivie d'effet.

Le moyen de surveillance 6 a été décrit fonctionnellement. Il peut être réalisé par tout moyen et selon toute technologie apte à mettre en oeuvre les fonctions décrites. Cependant, s'agissant d'une fonction de sécurité, influant sur une utilisation sûre du dispositif de traitement 1, l'invention met en place une réalisation câblée, au moyen d'un circuit électrique et/ou électronique afin d'offrir une meilleure fiabilité. Il est ainsi évité de recourir, même partiellement, à une technologie informatique.

Le moyen de surveillance 6 est construit autour d'un composant dédié, adapté à cette fonction de surveillance d'un courant indicatif d'une vitesse. Un tel composant est, par exemple, un composant de référence TC670, tel que produit par la société MICROCHIP.

Un tel composant 10 est représenté à la figure 2 dans un environnement illustratif. Ce composant 10 comprend 6 broches P1-P6. P4 reçoit une tension d'alimentation Vdd, ici +5V et P2 est connectée à une référence de tension GND. En relation avec un ventilateur 5 câblé fonctionnellement entre une tension d'alimentation +12V et une référence de tension, une image en tension du courant circulant dans le ventilateur 5 est produite au moyen d'un condensateur Csense. Cette tension image du courant est transmise au composant 10 via sa broche P5 / SENSE. Le composant 10 analyse cette tension SENSE afin de détecter une vitesse faible, inférieure à un seuil de vitesse. Lorsqu'une telle condition de défaut apparait, le composant 10 change l'état du signal de sortie ALERT présent sur la broche P5. Ce signal passe d'un état haut à un état bas en présence d'un défaut, afin d'être « fail-safe ». Ici de manière illustrative ce signal allume une del 11 / ALERT LED. Le composant 10 assure la fonction de persistance, en ce que l'état de défaut/alerte est maintenu suite à une détection de défaut. La broche P3 / CLEAR permet cependant de réarmer et est ici, par exemple, connectée à une sortie d'un microcontrôleur, tel qu'un microcontrôleur contenu dans l'unité de commande 8. Une broche P1 /THRESHOLD permet d'entrée une consigne de seuil de vitesse, au moyen d'une tension proportionnelle à la tension d'alimentation Vdd. Ceci est ici, par exemple réalisé au moyen du pont diviseur R2/R3 dont les valeurs de résistance relatives déterminent le seuil de vitesse.

Un tel composant 10 est préférentiellement utilisé pour surveiller un ventilateur 5. On utilise alors préférentiellement un tel composant 10 par ventilateur 5.

Tel que décrit, le dispositif a pour but de maintenir une température modérée au niveau de la tête laser 2 afin de garantir un fonctionnement sûr et correct du dispositif 1.

Cependant, les mesures mises en oeuvre ne sont pas certaines d'assurer le résultat. Il est en effet possible que la tête laser 2 atteigne malgré tout des températures plus élevées. Ceci est particulièrement préjudiciable pour un pyromètre présent dans le dispositif 1, à proximité de la tête laser 2. Le pyrométre est séparé de la tête laser 2 par un isolant thermique, préférentiellement une mousse.

## Revendications

1. Un dispositif (1) de traitement dermatologique comprenant une tête laser (2) apte à tirer un faisceau laser (3) en direction d'une zone cible (4) qui correspond à la plaie d'un patient qui comprend des tissus dermiques pour créer un échauffement précis et localisé afin d'en accélérer la cicatrisation, au moins un pyromètre apte à mesurer une température de la zone cible et au moins un ventilateur (5) apte à dissiper la chaleur dégagée par la tête laser (2), au moins un moyen d'inhibition (7) du faisceau laser, **caractérisé en ce** ledit au moins un pyromètre est à proximité de la tête laser; et en ce que le dispositif comprend encore :
• un isolant thermique séparant ledit au moins un pyromètre de la tête laser (2), et
• au moins un moyen de surveillance (6) câblé prenant la forme d'un circuit électrique et/ou électronique apte à détecter un défaut d'au moins un ventilateur (5) et à commander ledit au moins un moyen d'inhibition (7) du faisceau laser (3) lorsqu'un tel défaut est détecté, lequel au moins un moyen de surveillance (6) comprend un moyen d'observation apte à observer le courant de commande circulant dans ledit au moins un ventilateur (5) et à détecter un défaut lorsque la vitesse dudit au moins un ventilateur devient inférieure à un seuil de vitesse ou s'annule.

2. Le dispositif (1) selon la revendication 1, où la commande du moyen d'inhibition (7) est persistante.

3. Le dispositif (1) selon la revendication 1, comprenant un moyen de configuration apte à permettre la configuration du seuil de vitesse.

4. Le dispositif (1) selon l'une quelconque des revendications 1 à 3, où ledit au moins un moyen de surveillance (6) comprend encore un moyen d'alerte apte à envoyer une alerte à l'attention d'un opérateur lorsqu'un défaut est détecté.

5. Le dispositif (1) selon la revendication 1, où l'isolant thermique est une mousse.

6. Le dispositif (1) selon l'une quelconque des revendications 1 à 5, où ledit au moins un moyen de surveillance (6) est construit autour d'un composant dédié, adapté à cette fonction de surveillance d'un courant indicatif d'une vitesse.

## Patentansprüche

1. Vorrichtung (1) für dermatologische Behandlungen, einen Laserkopf (2) umfassend, geeignet, einen Laserstrahl (3) auf einen Zielbereich (4) zu werfen, der der Wunde eines Patienten entspricht, die Hautgewebe umfasst, um eine präzise und lokale Erwärmung zu erzeugen, um deren Vernarbung zu beschleunigen, mindestens ein Pyrometer, geeignet, eine Temperatur des Zielbereichs zu messen, und mindestens einen Ventilator (5), geeignet, die vom Laserkopf (2) abgegebene Wärme abzuführen, mindestens ein Mittel (7) zur Unterbrechung des Laserstrahls,
**dadurch gekennzeichnet, dass**
das genannte mindestens eine Pyrometer sich nahe dem Laserkopf befindet, und dadurch, dass die Vorrichtung außerdem umfasst
• eine Wärmeisolation, die das genannte mindestens eine Pyrometer vom Laserkopf (2) trennt, und
• mindestens ein verdrahtetes Überwachungsmittel (6) in Form eines elektrischen und/oder elektronischen Schaltkreises, geeignet, einen Fehler mindestens eines Ventilators (5) festzustellen und das genannten mindestens eine Mittel (7) zur Unterbrechung des Laserstrahls (3) zu betätigen, wenn ein derartiger Fehler festgestellt wird, wobei dieses mindestens eine Überwachungsmittel (6) ein Beobachtungsmittel umfasst, geeignet, den Steuerstrom zu beobachten, der im mindestens einen Ventilator (5) fließt, und einen Fehler festzustellen, wenn die Geschwindigkeit des genannten mindestens einen Ventilators einen Geschwindigkeitsschwellenwert unterschreitet oder auf Null abfällt.

2. Vorrichtung (1) nach Patentanspruch 1, in der die Betätigung des Unterbrechungsmittels (7) bleibend ist.

3. Vorrichtung (1) nach Patentanspruch 1, ein Konfiguriermittel umfassend, geeignet, das Konfigurieren des Geschwindigkeitsschwellenwertes zu erlauben.

4. Vorrichtung (1) nach irgendeinem der Patentansprüche 1 bis 3, in der das genannte mindestens eine Überwachungsmittel (6) außerdem ein Warnmittel umfasst, geeignet, einem Bediener eine Warnung zu senden, wenn ein Fehler festgestellt wird.

5. Vorrichtung (1) nach Patentanspruch 1, in der die Wärmeisolation ein Schaumstoff ist.

6. Vorrichtung (1) nach irgendeinem der Patentansprüche 1 bis 5, in der das genannte mindestens eine Überwachungsmittel (6) um eine dazu bestimmte Komponente gebaut ist, die an diese Funktion der Überwachung eines eine Geschwindigkeit angebenden Stromes angepasst ist.

## Claims

1. A dermatological treatment device (1) comprising a laser head (2) able to fire a laser beam (3) toward a target zone (4) corresponding to the wound of a patient that comprises dermal tissues to create precise and localized heating in order to accelerate healing, at least one pyrometer able to measure a temperature of the target zone, at least one fan (5) suitable for dissipating the heat emitted by the laser head (2), and at least one means (7) for inhibiting the laser beam (3), **characterized in that** the at least one pyrometer is near the laser head (2), **and in that** the device further comprises:
• A thermal insulator separating said at least one pyrometer from the laser head (2),
• At least one cabled monitoring means (6) being an electrical and/or electronic circuit suitable for detecting a fault in at least one fan (5) and controlling at least one means (7) for inhibiting the laser beam (3) when a fault is detected; said at least one monitoring means (6) comprising an observation means suitable to observe the command current circulating in the at least one fan (5), and detects a fault when the speed becomes lower than a speed threshold or is canceled out.

2. The device (1) according to claim 1, where the command of the inhibiting means (7) is persistent.

3. The device (1) according to claim 1, comprising a configuring means suitable for allowing the configuration of the speed threshold.

4. The device (1) according to any one of claims 1 to 3, where the at least one monitoring means (6) further comprises an alert means suitable for sending an alert to an operator when a fault is detected.

5. The device (1) according to claim 1, where the thermal insulator is a foam.

6. The device (1) according to any one of claims 1 to 5, where said monitoring means (6) is built around a dedicated component, suitable for this monitoring function of a current indicative of a speed.
